# EUROPÄISCHE PATENTSCHRIFT

(11) **EP 3 463 285 B1**
(45) Veröffentlichungstag und Bekanntmachung des Hinweises auf die Patenterteilung: **06.10.2021**
(21) Anmeldenummer: 17720062.3
(22) Anmeldetag: 24.04.2017
(51) Int. Cl.: A61K 8/81, A61K 8/58, A61Q 15/00

(54) **SCHWEISSHEMMENDE ZUBEREITUNG**
ANTIPERSPIRANT PREPARATION
PRÉPARATION ANTITRANSPIRANTE

(30) Priorität: 23.05.2016 DE 102016208860
(43) Veröffentlichungstag der Anmeldung: 10.04.2019
(73) Patentinhaber: Henkel AG & Co. KGaA, 40589 Düsseldorf (DE)
(72) Erfinder: DÖRING, Thomas, 41540 Dormagen (DE); TECKENBROCK, Gertraud, 45549 Sprockhövel (DE)
(86) Internationale Anmeldenummer: PCT/EP2017/059593
(87) Internationale Veröffentlichungsnummer: WO 2017/202556

(56) Entgegenhaltungen:
- WO-A1-93/24105
- WO-A1-2015/085998
- WO-A1-2016/062507

## Beschreibung

Die vorliegende Anmeldung betrifft kosmetische Antitranspirant-Zusammensetzungen mit verbesserter Wirkung und Verfahren unter Einsatz dieser Zusammensetzungen.

Die Reinigung und Pflege des eigenen Körpers schließt in zahlreichen Kulturkreisen Maßnahmen mit ein, die der Entstehung eines als unangenehm empfundenen übermäßigen Körpergeruchs entgegenwirken sollen. Die Entstehung des Körpergeruchs wird unter anderem durch die Transpiration gefördert, weshalb die kosmetische Industrie zahlreiche Körperpflegemittel bereitstellt, welche für die Anwendung in Körperregionen mit einer hohen Dichte von Schweißdrüsen, insbesondere im Achselbereich, entwickelt wurden. Diese Körperpflegemittel wirken schweißhemmend (Antitranspirant) und/oder desodorierend (Deodorant).

Kosmetische Antitranspirantien des Standes der Technik enthalten üblicher Weise neben mindestens einem Öl oder einer Fettsubstanz und einer Riechstoffkomponente bzw. einem Parfüm mindestens ein schweißhemmendes Salz. Die in diesen Antitranspirantien eingesetzten schweißhemmenden Salze verringern zum einen die Schweißsekretion des Körpers durch eine temporäre Verengung und/oder Verstopfung der Ausführungsgänge der Schweißdrüsen, so dass die Schweißmenge um etwa 20 bis 60 Prozent reduziert werden kann. Zum anderen weisen sie aufgrund ihrer antimikrobiellen Wirkung einen zusätzlichen desodorierenden Effekt auf. Als schweißhemmende Salze werden für gewöhnlich aktivierte basische Aluminium- und Aluminium-Zirkoniumhalogenide eingesetzt. Weiterhin können auch Aluminium- und Aluminium-Zirkoniumhalogenide eingesetzt werden, welche mit organischen Säuren als Komplexliganden stabilisiert sind. Menschen mit empfindlicher Haut können auf schweißhemmende Salze mit Hautreizungen reagieren. Die der Verwendung von Antitranspirantien auf Grundlage von Aluminium- und Aluminium-Zirkoniumhalogenide können sich zudem auf der behandelten Haut oder auf den mit der behandelten Haut in Kontakt stehenden Textilien sichtbare und im Falle von Textilien auch waschresistente Rückstände des kosmetischen Mittels bilden.

Aluminiumfreie Antitranspirantien und Deodorantien werden beispielsweise in den deutschen Patentanmeldungen DE102013225617 A1 und DE102013225620 A1 beschrieben.

Die internationale Anmeldung WO 93/24105 A1 beschreibt aluminiumfreie Antitranspirantien auf Grundlage unterschiedlicher wasserunlöslicher Polymere.

Diese und andere aluminiumfreie Antitranspirantien genügen jedoch nicht den gestiegenen kosmetischen Anforderungen.

Vor diesem Hintergrund bestand die technische Aufgabe der vorliegenden Anmeldung in der Bereitstellung eines Antitranspirants mit hoher Wirksamkeit gegen Körpergeruch, einer guten Hautverträglichkeit und einer deutlich verminderten Rückstandsbildung auf Haut und Textil. Das Antitranspirant sollte sich zudem durch eine hohe Stabilität auszeichnen.

Es wurde nun überraschend gefunden, dass die vorgenannten technischen Aufgaben durch aluminiumfreie Zusammensetzungen gelöst werden, welche neben einem spezifischen amphomeren Polymer weiterhin ein Öl enthalten. Durch die vorliegende Erfindung wird bereitgestellt:
1. Antitranspirantes kosmetisches Mittel, welches enthält:
   a) 0,5 bis 10 Gew.-% mindestens eines Copolymers aus den Monomeren
      i) N-tert-Octylacrylamid
      ii) Acrylsäure
      iii) tert.-Butylaminoethylmethacrylat
      iv) sowie gegebenenfalls weiteren Monomeren
   b) 40 bis 75 Gew.-% bei 20°C und 1.013 hPa flüssiges Öl
   wobei das antitranspirante kosmetische Mittel keine aluminiumhaltigen Verbindungen enthält.
2. Desodorierendes kosmetisches Mitteln nach Punkt 1, wobei das Mittel als Copolymer a) mindestens ein Copolymer aus den Monomeren
   i) N-tert-Octylacrylamid
   ii) Acrylsäure
   iii) tert.-Butylaminoethylmethacrylat
   iv) Methylmethacrylat
   v) Hydroxypropylmethacrylat
   enthält.
3. Desodorierendes kosmetisches Mittel nach einem der vorherigen Punkte, wobei das Copolymer a) bezogen auf das Gesamtgewicht des Mittels in einer Menge von 1,0 bis 10 Gew.-%, vorzugsweise von 3,0 bis 7,0 Gew.-% enthalten ist.
4. Desodorierendes kosmetisches Mittel nach einem der vorherigen Punkte, wobei das Öl ausgewählt ist aus der Gruppe Siliconöle, bevorzugt aus der Gruppe der Dialkyl- und Alkylarylsiloxane, insbesondere aus der Gruppe Cyclopentasiloxan, Cyclohexasiloxan, Dimethylpolysiloxan und Methylphenylpolysiloxan.
5. Desodorierendes kosmetisches Mittel nach einem der vorherigen Ansprüche, wobei als Öl Cyclopentasiloxan enthalten ist.
6. Desodorierendes kosmetisches Mittel nach einem der vorherigen Punkte, wobei das Öl, bezogen auf das Gesamtgewicht des Mittels, in einer Menge von 25 bis 70 Gew.-%, vorzugsweise von 35 bis 55 Gew.-% enthalten ist.
7. Desodorierendes kosmetisches Mittel nach einem der vorherigen Punkte, wobei das kosmetische Mittel, bezogen auf das Gesamtgewicht des Mittels, 10 bis 60 Gew.-%, vorzugsweise 15 bis 50 Gew.-% und insbesondere 20 bis 40 Gew.-% hydrophiles organisches Lösungsmittel enthält.
8. Desodorierendes kosmetisches Mittel nach Punkt 7, wobei das hydrophile organische Lösungsmittel ausgewählt ist aus der Gruppe Ethanol und Propylencarbonat.
9. Desodorierendes kosmetisches Mittel nach einem der vorherigen Punkte, wobei das kosmetische Mittel, bezogen auf das Gesamtgewicht des Mittels, 10 bis 50 Gew.-%, vorzugsweise 14 bis 46 Gew.-% und insbesondere 18 bis 42 Gew.-% Ethanol enthält.
10. Desodorierendes kosmetisches Mittel nach einem der vorherigen Punkte, wobei das kosmetische Mittel, bezogen auf das Gesamtgewicht des Mittels, 0,5 bis 4,0 Gew.-%, vorzugsweise 1,0 bis 3,5 Gew.-% und insbesondere 1,5 bis 3,0 Gew.-% Propylencarbonat enthält.
11. Desodorierendes kosmetisches Mittel nach einem der vorherigen Punkte, wobei das Mittel einen Dampfdruck (20°C) von 1,0 bis 3,0 kPa, bevorzugt von 1,2 bis 2,7 kPa und insbesondere von 1,4 bis 2,5 kPa aufweist.
12. Desodorierendes kosmetisches Mittel nach einem der vorherigen Punkte, wobei Wasser bezogen auf das Gesamtgewicht des Mittels in Mengen von weniger als 5,0 Gew,-%, vorzugsweise von weniger als 3,0 Gew.-% und insbesondere von weniger als 1,0 Gew.-% enthalten ist.
13. Desodorierendes kosmetisches Mittel nach einem der vorherigen Punkte, wobei das Mittel, bezogen auf das Gesamtgewicht des Mittels, 2,0 bis 20 Gew.-%, vorzugsweise 4,0 bis 16 Gew.-% und insbesondere 8,0 bis 12 Gew.-% mindestens eines Enzyminhibitors enthält.
14. Desodorierendes kosmetisches Mittel nach einem der vorherigen Punkte, wobei das Mittel einen Enzyminhibitor aus der Gruppe der Trialkylcitronensäureester, vorzugsweise Triethylcitrat oder Zinkglycinat, insbesondere Triethylcitrat enthält.
15. Desodorierendes kosmetisches Mittel nach einem der vorherigen Punkte, wobei das Mittel bezogen aus das Gesamtgewicht des Mittels 0,5 bis 10 Gew.-%, bevorzugt 1,0 bis 9,0 Gew.-% und insbesondere 4,0 bis 8,0 Gew.-% Verdickungsmittel enthält.
16. Desodorierendes kosmetisches Mittel nach einem der vorherigen Punkte, wobei das Mittel ein Verdickungsmittel aus der Gruppe der hydrophob modifizierten Silikate, bevorzugt aus der Gruppe der hydrophobierten Hectorite, insbesondere aus der Gruppe der hydrophobierten Hectorite mit der INCI-Bezeichnung Quaternium-18 Hectorite enthält.
17. Desodorierendes kosmetisches Mittel nach einem der vorherigen Punkte, wobei das kosmetische Mittel, bezogen auf das Gesamtgewicht des Mittels, zu mindestens 80 Gew.-%, vorzugweise zu mindestens 90 Gew.-% und insbesondere zu mindestens 95 Gew.-% aus
   a) mindestens einem Copolymers aus den Monomeren
      i) N-tert-Octylacrylamid
      ii) Acrylsäure
      iii) tert.-Butylaminoethylmethacrylat
      iv) sowie gegebenenfalls weiteren Monomeren
   b) bei 20°C und 1.013 hPa flüssigem Öl
   c) hydrophilem organischem Lösungsmittel
   d) Enzyminhibitor
   e) Verdickungsmittel
   besteht.
18. Kosmetische Zubereitung umfassend,
   i) desodorierendes kosmetisches Mittel nach einem der vorherigen Punkte
   ii) Treibmittel.
19. Kosmetische Zubereitung umfassend, bezogen auf ihr Gesamtgewicht,
   i) 10 bis 30 Gew.-% kosmetisches Mittel nach einem der vorherigen Punkte
   ii) 70 bis 90 Gew.-% Treibmittel.
20. Kosmetisches Produkt, umfassend
   - ein kosmetisches Mittel oder eine kosmetische Zubereitung nach einem der vorherigen Punkte
   - eine Abgabevorrichtung mit Sprühventil.
21. Verwendung eines desodorierenden kosmetischen Mittels, einer kosmetischen Zubereitung oder eines kosmetischen Produkts nach einem der vorherigen Punkte zur Verhinderung und/oder Reduzierung des Körpergeruchs.
22. Nicht-therapeutisches kosmetisches Verfahren zur Verhinderung und/oder Reduzierung des Körpergeruchs, bei welchem ein kosmetisches Mittel nach einem der vorherigen Punkte auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut verbleibt.

Ein erster zwingender Bestandteil der erfindungsgemäßen antitranspiranten kosmetischen Mittel ist das Copolymer a). In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des Copolymers a) am Gesamtgewicht des Mittels von 1,0 bis 10 Gew.-%, vorzugsweise von 3,0 bis 7,0 Gew.-% beträgt.

Das Copolymer a) ist auf die Monomere i) N-tert-Octylacrylamid, ii) Acrylsäure, iii) tert.-Butylaminoethylmethacrylat sowie gegebenenfalls weitere Monomere zurückführbar.

Bevorzugte Copolymere a) bestehen zu mindestens 90 Gew.-%, vorzugsweise zu mindestens 95 Gew.-% und insbesondere zu mindestens 97 Gew.-% oder gar vollständig aus den Monomeren N-tert-Octylacrylamid, Acrylsäure und tert.-Butylaminoethylmethacrylat..

Besonders bevorzugt sind Copolymere a) aus den Monomeren i) N-tert-Octylacrylamid, ii) Acrylsäure, iii) tert.-Butylaminoethylmethacrylat, iv) Methylmethacrylat und v) Hydroxypropylmethacrylat.

Die zuvor beschriebenen Copolymere a) werden beispielsweise unter der Bezeichnung Amphomer® (INCI-Bezeichnung: Octylacrylamide / Acrylates / Butylaminoethyl Methacrylate Copolymer; CAS Nummer 70801-07-9) von der Firma National Starch vertrieben.

Ein besonders bevorzugter Gegenstand der vorliegenden Patentanmeldung ist ein kosmetisches Mittel, umfassend mindestens ein Copolymer a) aus den Monomeren
i) N-tert-Octylacrylamid
ii) Acrylsäure
iii) tert.-Butylaminoethylmethacrylat
iv) Methylmethacrylat
v) Hydroxypropylmethacrylat.

Als zweiten wesentlichen Bestandteil b) enthalten die kosmetischen Mittel 40 bis 75 Gew.-% bei 20°C und 1.013 hPa flüssiges Öl. Mit dem Begriff "Öl" werden Substanzen bezeichnet, die sich nicht mit Wasser mischen.

Besonders bevorzugte kosmetische Mittel enthalten mindestens ein bei 20°C und 1.013 hPa flüssiges Öl aus der Gruppe der
- Ester von linearen oder verzweigten gesättigten oder ungesättigten C₂₋₃₀-Fettalkoholen mit linearen oder verzweigten gesättigten oder ungesättigten C₂₋₃₀-Fettsäuren,
- Anlagerungsprodukte von Ethylenoxid und/oder Propylenoxid an ein- oder mehrwertige C₃₋₂₂-Alkanole
- Dialkylether und
- Paraffine. Diese Öle haben sich in Verbindung mit dem Copolymer als besonders wirksam erwiesen.

Ganz besonders bevorzugt ist der Einsatz von Öl aus der Gruppe der Siliconöle, bevorzugt aus der Gruppe der Dialkyl- und Alkylarylsiloxane, insbesondere aus der Gruppe Cyclopentasiloxan, Cyclohexasiloxan, Dimethylpolysiloxan und Methylphenylpolysiloxan, wobei Cyclopentasiloxan insbesondere bevorzugt ist.

Desodorierendes kosmetisches Mittel nach einem der vorherigen Punkte, wobei das Öl, bezogen auf das Gesamtgewicht des Mittels, in einer Menge von 25 bis 70 Gew.-%, vorzugsweise von 35 bis 55 Gew.-% enthalten ist.

Die kosmetischen Mittel enthalten keine aluminiumhaltigen Verbindungen. Unter dem Begriff "aluminiumhaltige Verbindungen" werden im Rahmen der vorliegenden Erfindung schweißhemmende Aluminiumsalze und Aluminium-Zirkonium-Salze verstanden.

Bevorzugte kosmetische Mittel enthalten neben dem Öl als weiteren Träger hydrophiles organisches Lösungsmittel.

Eine besonders vorteilhafte kosmetische Wirkung konnte mit solchen Mittel erreicht werden, welche, bezogen auf ihr Gesamtgewicht, 10 bis 60 Gew.-%, vorzugsweise 15 bis 50 Gew.-% und insbesondere 20 bis 40 Gew.-% hydrophiles organisches Lösungsmittel enthalten.

Bevorzugte hydrophile organische Lösungsmittel sind ausgewählt aus der Gruppe Ethanol und Propylencarbonat.

Bei Ethanol haltigen kosmetischen Mittel beträgt der Ethanolgehalt am Gesamtgewicht des Mittels, vorzugsweise 10 bis 50 Gew.-%, bevorzugt 14 bis 46 Gew.-% und insbesondere 18 bis 42 Gew.-%. Dem gegenüber beträgt der Gewichtsanteil des Propylencarbonats am Gesamtgewicht bevorzugter kosmetischer Mittel 0,5 bis 4,0 Gew.-%, vorzugsweise 1,0 bis 3,5 Gew.-% und insbesondere 1,5 bis 3,0 Gew.-% Propylencarbonat.

Die kosmetischen Mittel werden vorzugsweise wasserarm oder wasserfrei formuliert. Bevorzugte desodorierende kosmetische Mittel enthalten, bezogen auf das Gesamtgewicht des Mittels, Wasser in Mengen von weniger als 5,0 Gew,-%, vorzugsweise von weniger als 3,0 Gew.-% und insbesondere von weniger als 1,0 Gew.-%.

Als ein für die kosmetische Wirkung der erfindungsgemäßen Mittel besonders relevanter technischer Parameter hat sich der Dampfdruck dieser Mittel erwiesen. Kosmetisch besonders vorteilhafte kosmetische Mittel weisen einen Dampfdruck (20°C) von 1,0 bis 3,0 kPa, bevorzugt von 1,2 bis 2,7 kPa und insbesondere von 1,4 bis 2,5 kPa auf.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 6 | Formel 7 | Formel 8 | Formel 9 | Formel 10 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 11 | Formel 12 | Formel 13 | Formel 14 | Formel 15 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 16 | Formel 17 | Formel 18 | Formel 19 | Formel 20 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 21 | Formel 22 | Formel 23 | Formel 24 | Formel 25 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 26 | Formel 27 | Formel 28 | Formel 29 | Formel 30 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 31 | Formel 32 | Formel 33 | Formel 34 | Formel 35 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 36 | Formel 37 | Formel 38 | Formel 39 | Formel 40 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 41 | Formel 42 | Formel 43 | Formel 44 | Formel 45 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 46 | Formel 47 | Formel 48 | Formel 49 | Formel 50 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 51 | Formel 52 | Formel 53 | Formel 54 | Formel 55 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 56 | Formel 57 | Formel 58 | Formel 59 | Formel 60 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 61 | Formel 62 | Formel 63 | Formel 64 | Formel 65 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 66 | Formel 67 | Formel 68 | Formel 69 | Formel 70 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 71 | Formel 72 | Formel 73 | Formel 74 | Formel 75 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 76 | Formel 77 | Formel 78 | Formel 79 | Formel 80 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 81 | Formel 82 | Formel 83 | Formel 84 | Formel 85 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * mit der INCI Bezeichnung Octylacrylamide / Acrylates / Butylaminoethyl Methacrylate Copolymer | | | | | |

Neben den zuvor beschriebenen Inhaltsstoffen können die desodorierenden kosmetischen Mittel weitere Wirk- und Hilfsstoffe enthalten. Zur Gruppe dieser weiteren Wirk- und Hilfsstoffe zählen beispielsweise die Enzyminhibitoren.

Geeignete Enzyminhibitoren sind beispielsweise Esteraseinhibitoren. Hierbei handelt es sich vorzugsweise um Trialkylcitrate wie Trimethylcitrat, Tripropylcitrat, Trii sopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispiels weise Lanosterin-, Cholesterin-, Campesterin-, Stigmasterin- und Sitosterin sulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat. Besonders bevorzugte Enzyminhibitoren sind ausgewählt aus der Gruppe der Trialkylcitronensäureester, vorzugsweise aus Triethylcitrat oder Zinkglycinat, insbesondere Triethylcitrat.

Bevorzugte Mittel enthalten, bezogen auf ihr Gesamtgewicht, 2,0 bis 20 Gew.-%, vorzugsweise 4,0 bis 16 Gew.-% und insbesondere 8,0 bis 12 Gew.-% Enzyminhibitor.

Die Zusammensetzung weiterer bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 101 | Formel 102 | Formel 103 | Formel 104 | Formel 105 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 106 | Formel 107 | Formel 108 | Formel 109 | Formel 110 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 111 | Formel 112 | Formel 113 | Formel 114 | Formel 115 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 116 | Formel 117 | Formel 118 | Formel 119 | Formel 120 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 121 | Formel 122 | Formel 123 | Formel 124 | Formel 125 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 126 | Formel 127 | Formel 128 | Formel 129 | Formel 130 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 131 | Formel 132 | Formel 133 | Formel 134 | Formel 135 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 136 | Formel 137 | Formel 138 | Formel 139 | Formel 140 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 141 | Formel 142 | Formel 143 | Formel 144 | Formel 145 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 146 | Formel 147 | Formel 148 | Formel 149 | Formel 150 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 151 | Formel 152 | Formel 153 | Formel 154 | Formel 155 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 156 | Formel 157 | Formel 158 | Formel 159 | Formel 160 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 161 | Formel 162 | Formel 163 | Formel 164 | Formel 165 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 166 | Formel 167 | Formel 168 | Formel 169 | Formel 170 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat | | | | | |

| | Formel 171 | Formel 172 | Formel 173 | Formel 174 | Formel 175 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 176 | Formel 177 | Formel 178 | Formel 179 | Formel 180 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise Triethylcitrat | | | | | |

| | Formel 181 | Formel 182 | Formel 183 | Formel 184 | Formel 185 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Enzyminhibitor ** | 2,0 bis 20 | 2,0 bis 20 | 4,0 bis 16 | 4,0 bis 16 | 8,0 bis 12 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * mit der INCI Bezeichnung Octylacrylamide / Acrylates / Butylaminoethyl Methacrylate Copolymer ** vorzugsweise Triethylcitrat | | | | | |

Eine weitere Gruppe optionaler Wirk- und Hilfsstoffe sind die Verdickungsmittel, welche zur Einstellung der Viskosität eingesetzt werden. Das Verdickungsmittel ist von den zwingend in den kosmetischen Mitteln enthaltenen Bestandteilen Copolymer und Öl verschieden. Ein besonders bevorzugtes Verdickungsmittel sind die hydrophob modifizierten Silikate, insbesondere die hydrophobierten Hectorite, besonders bevorzugt die hydrophobierten Hectorite der INCI-Bezeichnung Quaternium-18 Hectorite, wie sie beispielsweise unter dem Handelsnamen Bentone 38 (Elementis) vertrieben werden.

Bevorzugte kosmetische Mitteln enthalten das Verdickungsmittel, insbesondere das hydrophob modifizierte Silikate, besonders bevorzugte das hydrophobierte Hectorit in Mengen von 0,5 bis 10 Gew.-%, bevorzugt von 1,0 bis 9,0 Gew.-% und insbesondere von 4,0 bis 8,0 Gew.-%, jeweils bezogen auf das Gesamtgewicht des kosmetischen Mittels

In den folgenden Tabellen ist die Zusammensetzung weiterer bevorzugter kosmetischer Mittel dargestellt (Angaben in Gew.-% bezogen auf das Gesamtgewicht des kosmetischen Mittels sofern nicht anders angegeben).

| | Formel 201 | Formel 202 | Formel 203 | Formel 204 | Formel 205 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 206 | Formel 207 | Formel 208 | Formel 209 | Formel 210 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 211 | Formel 212 | Formel 213 | Formel 214 | Formel 215 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 216 | Formel 217 | Formel 218 | Formel 219 | Formel 220 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 221 | Formel 222 | Formel 223 | Formel 224 | Formel 225 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 226 | Formel 227 | Formel 228 | Formel 229 | Formel 230 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 231 | Formel 232 | Formel 233 | Formel 234 | Formel 235 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 236 | Formel 237 | Formel 238 | Formel 239 | Formel 240 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| hydrophiles organisches Lösungsmittel | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 241 | Formel 242 | Formel 243 | Formel 244 | Formel 245 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 246 | Formel 247 | Formel 248 | Formel 249 | Formel 250 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 251 | Formel 252 | Formel 253 | Formel 254 | Formel 255 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 256 | Formel 257 | Formel 258 | Formel 259 | Formel 260 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 261 | Formel 262 | Formel 263 | Formel 264 | Formel 265 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 266 | Formel 267 | Formel 268 | Formel 269 | Formel 270 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 271 | Formel 272 | Formel 273 | Formel 274 | Formel 275 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren ** vorzugsweise hydrophobierter Hectorit, insbesondere hydrophobierter Hectorit mit der INCI-Bezeichnung Quaternium-18 Hectorite | | | | | |

| | Formel 276 | Formel 277 | Formel 278 | Formel 279 | Formel 280 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| aus den Monomeren i) N-tert-Octylacrylamid, ii) Acrylsäure, iii) tert.-Butylaminoethylmethacrylat, iv) Methylmethacrylat und v) Hydroxypropylmethacrylat ** vorzugsweise hydrophobierter Hectorit, insbesondere hydrophobierter Hectorit mit der INCI-Bezeichnung Quaternium-18 Hectorite | | | | | |

| | Formel 281 | Formel 282 | Formel 283 | Formel 284 | Formel 285 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Verdickungsmittel ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * mit der INCI Bezeichnung Octylacrylamide / Acrylates / Butylaminoethyl Methacrylate Copolymer ** vorzugsweise hydrophobierter Hectorit, insbesondere hydrophobierter Hectorit mit der INCI-Bezeichnung Quaternium-18 Hectorite | | | | | |

Kosmetische Mittel, welche größtenteils aus Copolymer, Öl, hydrophilem organischem Lösungsmittel, Enzyminhibitor und Verdickungsmittels bestehen, haben sich als sehr wirkungsvoll erwiesen. Aus Gründen der kosmetischen Wirkung und mit dem Ziel, die Komplexität der Herstellung dieser Mittel zu minimieren, ist es daher bevorzugt, dass das desodorierende kosmetische Mittel, bezogen auf sein Gesamtgewicht, zu mindestens 80 Gew.-%, vorzugweise zu mindestens 90 Gew.-% und insbesondere zu mindestens 95 Gew.-% aus
a) mindestens einem Copolymers aus den Monomeren
   i) N-tert-Octylacrylamid
   ii) Acrylsäure
   iii) tert.-Butylaminoethylmethacrylat
   iv) sowie gegebenenfalls weiteren Monomeren
b) bei 20°C und 1.013 hPa flüssigem Öl
c) hydrophilem organischem Lösungsmittel
d) Enzyminhibitor
e) Verdickungsmittel
besteht.

Die Zusammensetzung weiterer ganz besonders bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 401 | Formel 402 | Formel 403 | Formel 404 | Formel 405 |
|---|---|---|---|---|---|
| Copolymer * | 0,5 bis 10 | 0,5 bis 10 | 1,0 bis 10 | 1,0 bis 10 | 3,0 bis 7,0 |
| flüssiges Öl (20°C, 1.013 hPa) | 40 bis 75 | 25 bis 70 | 25 bis 70 | 25 bis 70 | 35 bis 55 |
| Enzyminhibitor | 2,0 bis 20 | 2,0 bis 20 | 4,0 bis 16 | 4,0 bis 16 | 8,0 bis 12 |
| Verdickungsmittel | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * aus den Monomeren i) N-tert-Octylacrylamid, ii) Acrylsäure, iii) tert.-Butylaminoethylmethacrylat sowie gegebenenfalls weiteren Monomeren | | | | | |

| | Formel 401 | Formel 402 | Formel 403 | Formel 404 | Formel 405 |
|---|---|---|---|---|---|
| Copolymer * | 0,5 bis 10 | 0,5 bis 10 | 1,0 bis 10 | 1,0 bis 10 | 3,0 bis 7,0 |
| Cyclopentasiloxan | 40 bis 75 | 25 bis 70 | 25 bis 70 | 25 bis 70 | 35 bis 55 |
| Triethylcitrat | 2,0 bis 20 | 2,0 bis 20 | 4,0 bis 16 | 4,0 bis 16 | 8,0 bis 12 |
| hydrophobierter Hectorit ** | 0,5 bis 10 | 1,0 bis 9,0 | 1,0 bis 9,0 | 1,0 bis 9,0 | 4,0 bis 8,0 |
| Ethanol | 10 bis 60 | 15 bis 50 | 15 bis 50 | 20 bis 40 | 20 bis 40 |
| Wasser | < 5,0 | < 3,0 | < 3,0 | < 1,0 | < 1,0 |
| optionale Additive | ad 100 | ad 100 | ad 100 | ad 100 | ad 100 |

| | | | | | |
|---|---|---|---|---|---|
| * mit der INCI Bezeichnung Octylacrylamide / Acrylates / Butylaminoethyl Methacrylate Copolymer ** mit der INCI- Bezeichnung Quaternium-18 Hectorite | | | | | |

Die Applikation des desodorierenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem desodorierenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Bevorzugt ist jedoch der Einsatz eines Treibmittels. Bevorzugte kosmetische Zubereitungen umfassen daher neben dem kosmetischen Mittel i) weiterhin mindestens ein Treibmittel ii).

Geeignete Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Bevorzugte kosmetische Mittel sind dadurch gekennzeichnet, dass das Mittel mit mindestens einem Treibmittel b) aus der Gruppe der Gemische aus Propan und Butan und Dimethylether kombiniert wird.

Bevorzugte kosmetische Zubereitungen umfassen, bezogen auf ihr Gesamtgewicht
i) 10 bis 30 Gew.-% kosmetisches Mittel nach einem der vorherigen Punkte
ii) 70 bis 90 Gew.-% Treibmittel.

Als Druckgasbehälter für Aerosolanwendungen kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der kosmetischen Mittel a).

Sollen die erfindungsgemäßen Mittel aufgesprüht werden, werden diese Mittel vorteilhafterweise mit einer Abgabevorrichtung und einem Sprühventil versehen. Die resultierenden kosmetischen Produkte umfassen demgemäß ein erfindungsgemäßes kosmetisches Mittel oder eine erfindungsgemäße kosmetische Zubereitung sowie eine Abgabevorrichtung mit Sprühventil.

Ein weiterer Anspruchsgegenstand ist ein nicht-therapeutisches kosmetisches Verfahren zur Verhinderung und/oder Reduzierung des Körpergeruchs, bei welchem ein erfindungsgemäßes kosmetisches Mittel auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut verbleibt.

Wie eingangs ausgeführt haben die erfindungsgemäßen kosmetischen Mittel einen vorteilhaften Einfluss auf die Bildung des Körpergeruchs. Die Verwendung dieser kosmetischen Mittels zur Verhinderung und/oder Reduzierung des Körpergeruchs ist ein weiterer Gegenstand der vorliegenden Anmeldung.

### Beispiele

Die folgenden versprühbaren Antitranspirant Suspensionen wurden hergestellt (Angaben in Gew.-%):

| | **1** | **2** | **3** |
|---|---|---|---|
| Cyclopentasiloxan | ad 100 | ad 100 | ad 100 |
| Zitronensäuretriethylester | 10,0 | 10,0 | 10,0 |
| Ethanol | 20,5 | 20,5 | 35,0 |
| Amphomer ¹⁾ | 1,5 | 1,0 | 5,0 |
| Bentone 38 V CG ²⁾ | 6,0 | 6,0 | 6,0 |
| Propylencarbonat | 2,0 | 2,0 | 2,0 |
| Natriumhydroxid | 0,005 | 0,005 | 0,005 |

| | | | |
|---|---|---|---|
| ¹⁾ INCI-Bezeichnung: Octylacrylamide / Acrylates / Butylaminoethyl Methacrylate Copolymer ²⁾ INCI-Bezeichnung Quaternium-18 Hectorite | | | |

Die Rezepturen wurden im Gewichtsverhältnis von 1:4 mit dem Treibmittel Propan/Butan (15/85) in Aerosoldosen abgefüllt.

## Patentansprüche

1. Antitranspirantes kosmetisches Mittel, welches enthält:
a) 0,5 bis 10 Gew.-% mindestens eines Copolymers aus den Monomeren
i) N-tert-Octylacrylamid
ii) Acrylsäure
iii) tert.-Butylaminoethylmethacrylat
iv) sowie gegebenenfalls weiteren Monomeren
b) 40 bis 75 Gew.-% bei 20°C und 1.013 hPa flüssiges Öl
wobei das antitranspirante kosmetische Mittel keine aluminiumhaltigen Verbindungen enthält.

2. Desodorierendes kosmetisches Mitteln nach Anspruch 1, wobei das Mittel als Copolymer a) mindestens ein Copolymer aus den Monomeren
i) N-tert-Octylacrylamid
ii) Acrylsäure
iii) tert.-Butylaminoethylmethacrylat
iv) Methylmethacrylat
v) Hydroxypropylmethacrylat
enthält.

3. Desodorierendes kosmetisches Mittel nach einem der vorherigen Ansprüche, wobei das Copolymer a) bezogen auf das Gesamtgewicht des Mittels in einer Menge von 1,0 bis 10 Gew.-%, vorzugsweise von 3,0 bis 7,0 Gew.-% enthalten ist.

4. Desodorierendes kosmetisches Mittel nach einem der vorherigen Ansprüche, wobei als Öl Cyclopentasiloxan enthalten ist.

5. Desodorierendes kosmetisches Mittel nach einem der vorherigen Ansprüche, wobei das Öl, bezogen auf das Gesamtgewicht des Mittels, in einer Menge von 25 bis 70 Gew.-%, vorzugsweise von 35 bis 55 Gew.-% enthalten ist.

6. Desodorierendes kosmetisches Mittel nach einem der vorherigen Ansprüche, wobei das kosmetische Mittel, bezogen auf das Gesamtgewicht des Mittels, 10 bis 60 Gew.-%, vorzugsweise 15 bis 50 Gew.-% und insbesondere 20 bis 40 Gew.-% hydrophiles organisches Lösungsmittel enthält.

7. Kosmetische Zubereitung umfassend,
i) desodorierendes kosmetisches Mittel nach einem der vorherigen Ansprüche
ii) Treibmittel.

8. Kosmetisches Produkt, umfassend
- ein kosmetisches Mittel oder eine kosmetische Zubereitung nach einem der vorherigen Ansprüche
- eine Abgabevorrichtung mit Sprühventil.

9. Verwendung eines desodorierenden kosmetischen Mittels, einer kosmetischen Zubereitung oder eines kosmetischen Produkts nach einem der vorherigen Ansprüche zur Verhinderung und/oder Reduzierung des Körpergeruchs.

10. Nicht-therapeutisches kosmetisches Verfahren zur Verhinderung und/oder Reduzierung des Körpergeruchs, bei welchem ein kosmetisches Mittel nach einem der vorherigen Ansprüche auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut verbleibt.

## Claims

1. An antiperspirant cosmetic agent containing:
a) 0.5 to 10 wt.% of at least one copolymer of the monomers
i) N-tert-octylacrylamide
ii) acrylic acid
iii) tert-butylaminoethyl methacrylate
iv) and optionally further monomers
b) 40 to 75 wt.% of an oil which is liquid at 20 °C and 1,013 hPa
wherein the antiperspirant cosmetic agent does not contain any aluminum-containing compounds.

2. The deodorizing cosmetic agent according to claim 1, wherein the agent contains, as copolymer a), at least one copolymer of the monomers
i) N-tert-octylacrylamide
ii) acrylic acid
iii) tert-butylaminoethyl methacrylate
iv) methyl methacrylate
v) hydroxypropyl methacrylate.

3. The deodorizing cosmetic agent according to one of the preceding claims, wherein the copolymer a) is contained in an amount of from 1.0 to 10 wt.%, preferably from 3.0 to 7.0 wt.%, based on the total weight of the agent.

4. The deodorizing cosmetic agent according to one of the preceding claims, wherein cyclopentasiloxane is contained as an oil.

5. The deodorizing cosmetic agent according to one of the preceding claims, wherein the oil is contained in an amount of from 25 to 70 wt.%, preferably from 35 to 55 wt.%, based on the total weight of the agent.

6. The deodorizing cosmetic agent according to one of the preceding claims, wherein the cosmetic agent contains 10 to 60 wt.%, preferably 15 to 50 wt.% and in particular 20 to 40 wt.% hydrophilic organic solvent, based on the total weight of the agent.

7. A cosmetic preparation comprising
i) the deodorizing cosmetic agent according to one of the preceding claims
ii) a blowing agent.

8. A cosmetic product comprising
- a cosmetic agent or a cosmetic preparation according to one of the preceding claims
- a dispensing device having a spray valve.

9. The use of a deodorizing cosmetic agent, a cosmetic preparation or a cosmetic product according to one of the preceding claims for preventing and/or reducing body odor.

10. A non-therapeutic cosmetic method for preventing and/or reducing body odor, in which a cosmetic agent according to one of the preceding claims is applied to the skin, in particular to the skin of the armpits, and remains on the skin for at least 1 hour, preferably for at least 2 hours, preferably for at least 4 hours, in particular for at least 6 hours.

## Revendications

1. Agent cosmétique antitranspirant, contenant :
a) 0,5 à 10 % en poids d'au moins un copolymère provenant des monomères
i) de N-tert-octylacrylamide
ii) d'acide acrylique
iii) de méthacrylate de tert-butylaminoéthyle
iv) et éventuellement d'autres monomères
b) 40 à 75 % en poids d'huile liquide à 20 °C et 1 013 hPa
l'agent cosmétique antitranspirant ne contenant aucun composé contenant de l'aluminium.

2. Agent cosmétique désodorisant selon la revendication 1, l'agent contenant, en tant que copolymère a), au moins un copolymère provenant des monomères
i) de N-tert-octylacrylamide
ii) d'acide acrylique
iii) de méthacrylate de tert-butylaminoéthyle
iv) de méthacrylate de méthyle
v) de méthacrylate d'hydroxypropyle.

3. Agent cosmétique désodorisant selon l'une des revendications précédentes, dans lequel le copolymère a) est contenu en une quantité de 1,0 à 10 % en poids, de préférence de 3,0 à 7,0 % en poids, par rapport au poids total de l'agent.

4. Agent cosmétique désodorisant selon l'une des revendications précédentes, dans lequel l'huile contenue est le cyclopentasiloxane.

5. Agent cosmétique désodorisant selon l'une des revendications précédentes, dans lequel l'huile est contenue en une quantité de 25 à 70 % en poids, de préférence de 35 à 55 % en poids, par rapport au poids total de l'agent.

6. Agent cosmétique désodorisant selon l'une des revendications précédentes, dans lequel l'agent cosmétique contient 10 à 60 % en poids, de préférence 15 à 50 % en poids et en particulier 20 à 40 % en poids de solvant organique hydrophile, par rapport au poids total de l'agent.

7. Préparation cosmétique comprenant
i) un agent cosmétique désodorisant selon l'une des revendications précédentes
ii) un agent propulseur.

8. Produit cosmétique, comprenant
- un agent cosmétique ou une préparation cosmétique selon l'une des revendications précédentes
- un dispositif de distribution comportant une valve de pulvérisation.

9. Utilisation d'un agent cosmétique désodorisant, d'une préparation cosmétique ou d'un produit cosmétique selon l'une des revendications précédentes, pour prévenir et/ou réduire les odeurs corporelles.

10. Procédé cosmétique non thérapeutique permettant de prévenir et/ou de réduire les odeurs corporelles, dans lequel un agent cosmétique selon l'une des revendications précédentes est appliqué sur la peau, en particulier sur la peau des aisselles, et reste sur la peau pendant au moins 1 heure, de préférence pendant au moins 2 heures, préférablement pendant au moins 4 heures, en particulier pendant au moins 6 heures.
